# EUROPÄISCHE PATENTSCHRIFT

(11) **EP 2 913 066 B1**
(45) Veröffentlichungstag und Bekanntmachung des Hinweises auf die Patenterteilung: **26.07.2017**
(21) Anmeldenummer: 15156801.1
(22) Anmeldetag: 26.02.2015
(51) Int. Cl.: A61L 2/08, G21K 5/00, B65B 43/50, B65B 55/08

(54) **Vorrichtung und Verfahren zum Sterilisieren von Behältnissen**
Method and device for sterilising containers
Dispositif et procédé destinés à la stérilisation de récipients

(30) Priorität: 26.02.2014 DE 102014102537
(43) Veröffentlichungstag der Anmeldung: 02.09.2015
(73) Patentinhaber: Krones AG, 93073 Neutraubling (DE)
(72) Erfinder: Knott, Josef, 93073 Neutraubling (DE); Scheuren, Hans, 93073 Neutraubling (DE); Krüger, Jochen, 93073 Neutraubling (DE)
(74) Vertreter: Bittner, Bernhard

(56) Entgegenhaltungen:
- EP-A1- 1 403 186
- EP-A1- 1 956 608
- EP-A1- 2 151 510
- WO-A1-2008/034512

## Beschreibung

Die vorliegende Erfindung bezieht sich auf eine Vorrichtung und ein Verfahren zum Sterilisieren von Behältnissen. Derartige Vorrichtungen und Verfahren sind aus dem Stand der Technik seit längerer Zeit bekannt. In jüngerer Zeit ist man dazu bestrebt, nach Möglichkeit auf den Einsatz von chemischen Sterilisationsmitteln zu verzichten oder diesen zumindest zu reduzieren. Daher sind in jüngerer Zeit auch Sterilisationseinrichtungen bekannt geworden, welche die Wandungen der zu sterilisierenden Behältnisse mit einer Strahlung und insbesondere einer Ladungsträgerstrahlung beaufschlagen. Die vorliegende Erfindung wird im Wesentlichen unter Bezugnahme auf Elektronenstrahler beschrieben, es ist jedoch darauf hinzuweisen, dass eine Anwendung der Erfindung ggf. auch bei anderen Ladungsträgern denkbar ist, wie beispielsweise Protonen oder Alphateilchen. Daneben könnten auch andere Strahlungsarten, wie Röntgen oder UV-Strahlung, eingesetzt werden.

EP1956608 offenbart eine Plasma-Desinfektionsvorrichtung mit höhenverstellbarer Grundplatte.

Aus dem Stand der Technik sind Vorrichtungen und Verfahren bekannt, bei denen in die zu sterilisierenden Behältnisse über deren Mündungen Strahlfinger eingeführt werden. Diese Strahlfinger weisen dabei üblicherweise ein Austrittsfenster für Ladungsträger, wie insbesondere Elektronen, auf. Vorteilhaft ist dabei dieses Austrittsfenster an einer Unterseite der jeweiligen Strahlfinger angeordnet. Diese Strahlfinger müssen dabei einerseits einen Querschnitt aufweisen, der durch die Mündung in das Behältnis einführbar ist. Auf der anderen Seite müssen diese Strahlfinger in ihrem Inneren üblicherweise auch ein Vakuum aufrechterhalten, innerhalb dessen die Ladungsträger beschleunigt werden können. Dies führt dazu, dass derartige Strahlfinger oftmals sehr filigrane und auch kostenintensive Geräte sind. Aufgrund der geringen mechanischen Beanspruchbarkeit sind auch die Kosten bei einer einmaligen leichten oder auch schweren Beschädigung sehr hoch.

Weiterhin weisen Sterilisationseinrichtungen, welche zur Sterilisation eine Strahlung und insbesondere Elektronenstrahlen einsetzen, üblicherweise eine Elektronenerzeugungseinrichtung auf, sowie auch eine Beschleunigungseinrichtung, welche die Ladungsträger (insbesondere innerhalb eines/des Vakuums) in Richtung des Austrittsfensters beschleunigt.

Ebenso sind Vorrichtungen aus dem Stand der Technik bekannt, bei welchen die Sterilisationseinrichtungen an einem beweglichen Karussell angeordnet sind. Auch die Behältnisse und insbesondere Vorformlinge sind über Klammern an einem beweglichen Karussell angeordnet. Zur Innensterilisation wird jedes einzelne Behältnis an den Klammern über die Sterilisationseinrichtung bzw. den zu der Sterilisationseinrichtungen gehörenden Strahlenfinger bewegt. Dabei steht zur Erzeugung der Bewegung der jeweiligen Klammer jeweils ein Elektromotor (z. B. an dem Karussell) zur Verfügung, der individuell während des Behandlungszyklus ein Fahrprofil abarbeitet.

Nachteilig ist bei dem Stand der Technik jedoch, dass Linearmotoren sehr teuer sind und der Aufwand der Steuerung und Programmierung (beispielsweise zur Erzeugung des Fahrprofils) enorm ist, jedoch müssen bei einer Formatänderung der Behältnisse keine mechanischen Teile gewechselt werden.

Der vorliegenden Erfindung liegt daher die Aufgabe zugrunde, diese Nachteile zu beheben und eine Vorrichtung sowie ein Verfahren zu schaffen, was gleichzeitig kostengünstig und dennoch flexibel einsetzbar bzw. abänderbar ist und für die Sterilisation von verschiedensten Formaten von Behältnissen verwendet werden kann.

Dabei sollte jedoch die Mündung des Behältnisses im Wesentlichen gleich bleiben oder zumindest einen bestimmten minimalen Querschnitt aufweisen, da der stangenartige Körper bzw. Strahlenfinger noch sicher in das Behältnis geführt werden können muss, sodass durch die Vorrichtung Behältnisse mit verschiedenen Längen und Durchmessern kostengünstig sterilisiert werden können.

Eine erfindungsgemäße Vorrichtung zum Sterilisieren von Behältnissen ist in Anspruch 1 definiert. Somit erzeugt bevorzugt die Hubkurve eine Bewegung und besonders bevorzugt lediglich eine Hubbewegung in Längsrichtung L, der Behältnisse, durch welche die Behältnisse über den stangenartigen Körper bzw. Strahlenfinger geführt/bewegt werden bzw. führbar/bewegbar sind. Die Einführung des Strahlenfingers in die Behältnisse erfolgt daher durch eine Bewegung und bevorzugt nur durch eine Bewegung der Behältnisse, wobei diese Bewegung (die Hubbewegung in Längsrichtung L) bevorzugt lediglich durch die Hubkurve hervorgerufen und/oder vorgegeben wird. Die Hubkurve ist besonders bevorzugt eine feste Hubkurve, deren Verlauf ganz besonders bevorzugt nicht verändert werden kann. Dabei laufen die Klammern über diese Hubkurven, wodurch ein festes Fahr- bzw. Bewegungsprofil erreicht wird.

Unter einem überführen bzw. überbewegen wird dabei bevorzugt verstanden, dass der stangenartige Körper wenigstens abschnittsweise in das Behältnis bzw. den Innenraum/das Innere des Behältnisses eingeführt/eingebracht wird, wobei dieses Einführen/Einbringen bevorzugt durch eine Bewegung des Behältnisses erzeugt wird und nicht durch eine Bewegung der Sterilisationseinrichtung und/oder des stangenartigen Körpers. Weiterhin kann bevorzugt darunter eine Führen bzw. Bewegen des Behältnisses über den stangenartigen Körper von unterhalb des stangenartigen Körpers verstanden werden (bzw. Behältnisöffnung zeigt nach oben, vom Erdmittelpunkt weg), wobei aber auch eine Bewegung von oben und über den stangenartigen Körper möglich wäre (bzw. Behältnisöffnung zeigt nach unten, zum Erdmittelpunkt). Es wären aber auch andere Anordnungen bzw. Ausrichtungen, welche somit schräg verlaufen würden, denkbar. Dabei erstrecken sich die entsprechenden Elemente jedoch weiterhin in Längsrichtung L, es wird im Wesentlichen lediglich diese Längsrichtung L verändert, oder die Bewegungsrichtung entlang dieser Längsrichtung L.

Erfindungsgemäß weist die Vorrichtung eine Bewegungseinrichtung auf bzw. ist eine Bewegungseinrichtung vorgesehen, welche derart ausgeführt und ausgestaltet ist, dass eine Relativbewegung, in der Längsrichtung L der Behältnisse, zwischen dem stangenartigem Körper und der Hubkurve erzeugbar und ein Relativabstand r zwischen der Hubkurve und dem stangenartigen Körper veränderbar ist.

Daher wird vorgeschlagen das Fahr- bzw. Bewegungsprofil der Behältnisse durch die Hubkurve zu realisieren oder hervorzurufen, was zwar weniger flexibel ist, aber wesentlich kostengünstiger und für die meisten Verwendungszwecke der Vorrichtung ausreichend sein dürfte. Aufgrund der weiteren Relativbewegungsmöglichkeit, hervorgerufen durch die Bewegungseinrichtung, kann zusätzlich weiterhin einfach gewährleistet werden, dass auch verschieden lange Behältnisse bzw. Vorformlinge durch die Vorrichtung sterilisiert werden können, ohne große Umbauten/Änderungen vornehmen zu müssen.

Hinsichtlich der Hubkurven ergibt sich das Problem, dass ein festes Fahrprofil vorgegeben ist, sodass insbesondere kürzere Behältnisse nicht behandelt werden können, da diese (insbesondere deren Boden) mit dem Strahlenfinger kollidieren würde. Dieses Problem wird durch eine Veränderung des Relativabstands r zwischen der Hubkurve und dem stangenartigen Körper/Strahlenfinder gelöst.

Bevorzugt handelt es sich bei den Behältnissen um Kunststoffbehältnisse wie beispielsweise Kunststoffflaschen oder Kunststoffvorformlinge. Besonders bevorzugt handelt es sich bei den Behältnissen um Kunststoffvorformlinge.

Bei einer bevorzugten Ausführungsform ist die Bewegungseinrichtung derart ausgeführt und ausgestaltet, dass ein vorbestimmter Abstand a zwischen einem Bodenbereich der Behältnisse und dem stangenartigen Körper bzw. der Sterilisationseinrichtung einstellbar ist. Somit ist bevorzugt die Bewegungseinrichtung derart ausgeführt und ausgestaltet, dass eine Relativbewegung in der Längsrichtung L der Behältnisse zwischen dem stangenartigen Körper bzw. der Sterilisationseinrichtung und der Hubkurve erzeugbar und ein Relativabstand r zwischen der Hubkurve und dem stangenartigen Körper bzw. der Sterilisationseinrichtung veränderbar ist, derart, dass ein vorbestimmter Abstand a zwischen einem Bodenbereich der Behältnisse und dem stangenartigen Körper einstellbar ist.

Vorzugsweise ist der Abstand zwischen dem Bodenbereich der Behältnisse und dem stangenartigen Körper außerhalb eines Arbeitsbetriebs einstellbar.

Demnach wird bevorzugt die Relativbewegung in Längsrichtung L zwischen dem stangenartigen Körper bzw. der Sterilisationseinrichtung und der Hubkurve dazu verwendet, um einen vorbestimmten Abstand a zwischen Bodenbereich des Behältnisses und dem stangenartigen Körper auch bei verschieden langen Behältnissen einstellen zu können.

Der Abstand a wird dabei bevorzugt zwischen einem Mittelpunkt des Bodenbereichs, welcher bevorzugt im Wesentlichen im Bereich des Anspritzpunkts liegt, und dem stangenartigen Körper (insbesondere einem/dem Austrittsfenster des stangenartigen Körpers) erfasst, bestimmt, gemessen, ermittelt und/oder vorgegeben. Dieser Punkt, an welchem der Abstand a in Längsrichtung L (des Behältnisses) vom Bodenbereich des Behältnisses und dem Austrittsfenster ermittelt bzw. angesetzt wird, befindet sich daher bevorzugt an dem Punkt an dem sich eine Symmetrieachse S (diese erstreckt sich in Längsrichtung L des Behältnisses) des Behältnisses mit dem Bodenbereich schneidet. Dabei wird im Wesentlichen der innen liegendste Punkt des Bodens herangezogen. Es wäre generell aber auch die Verwendung eines anderer Punkts des Bodenbereichs möglich, um den Abstand a festzulegen bzw. durch welchen der Abstand a beschrieben ist oder wird.

Durch die Verwendung einer Hubkurve werden die Behältnisse und insbesondere deren Mündungen immer um einen vorbestimmten Hub/Weg in Längsrichtung L und über die Strahlenfinger bewegt. Wird die Produktion umgestellt und andere Behältnisse verwendet, welche kürzer sind (deren Ausdehnung in Längsrichtung L geringer ist) würde bei vorbestimmtem Hub/Weg der Bodenbereich der Behältnisse näher an den Strahlenfinger bzw. dessen Ende heranrücken oder diesen sogar berühren was zu erheblichen Schäden und Kosten führen würde. Durch die Bewegungseinrichtung kann nun zusätzlich der Relativabstand r zwischen dem Strahlenfinger bzw. dessen Ende und der Hubkurve angepasst werden, sodass beim Verwenden von kürzeren Behältnissen der Relativabstand r vergrößert werden kann, wodurch wiederum der vorbestimmte Abstand a zwischen dem Strahlenfinger bzw. dessen Ende und dem Bodenbereich eingestellt werden kann oder einstellbar ist.

Bevorzugt liegt der vorbestimmte Abstand a vor, wenn die Behältnisse den höchsten Punkt der Hubkurve abfahren bzw. sich auf diesem befinden. Bei dem höchsten Punkt kann es sich auch um einen längeren Bereich (somit einen höchsten Bereich) handeln. Als höchster Punkt ist hierbei im Wesentlichen der Punkt/Bereich zu verstehen, in dem die Hubkurve bzw. Bereiche/Teile der Hubkurve den geringsten Abstand zu dem Strahlenfinger und/oder der Sterilisationseinrichtung aufweist/aufweisen. Demnach ist der höchste Punkt im Umkehrpunkt vorliegend, in welchem die Behältnisse von einer Führung über den stangenartigen Körper/Strahlenfinger in eine umgekehrte Bewegung, in welcher die Behältnisse von dem Strahlenfinger abgezogen werden, übergeführt werden. Bevorzugt berühren sich der Strahlenfinger und die Behältnisse nie, sodass unter "abziehen" das Entfernen des Strahlenfingers aus dem Behältnis zu verstehen ist. Dieser Umkehrpunkt kann auch über einen bestimmten Bereich vorliegend sein bzw. die Hubkurve in diesem Bereich im Wesentlichen horizontal verlaufen. Beispielsweise wird zur Sterilisierung das Behältnis über den Strahlenfinger geführt, bis der vorbestimmte Abstand a erreicht ist, anschließend dieser Abstand zur Sterilisierung eine gewisse Zeit gehalten und anschließend das Behältnis wieder von dem Strahlenfinger abgezogen. Dies wird durch eine Erhebung, welche über einen bestimmten Bereich (oder sich über eine bestimmte Länge erstreckt, welche von der Geschwindigkeit eines Transports der Behältnisse abhängig ist) vorliegt, auf der Hubkurve erzeugt.

Bei einer weiteren bevorzugten Ausführungsform ist die Sterilisationseinrichtung und/oder der stangenartige Körper durch die Bewegungseinrichtung in der Längsrichtung L der Behältnisse bewegbar und die Hubkurve unbeweglich angeordnet. Somit ergibt sich bevorzugt, dass die erzeugte Relativbewegung in der Längsrichtung L zwischen dem stangenartigen Körper und der Hubkurve durch eine Bewegung der Sterilisationseinrichtung und/oder dem stangenartigen Körper erzeugt wird. Daher ist die Bewegungseinrichtung bevorzugt derart ausgeführt und ausgestaltet, dass die (Relativ-)Bewegung in der Längsrichtung L der Behältnisse zwischen dem stangenartigen Körper und der Hubkurve durch eine Bewegung der Sterilisationseinrichtung und/oder des stangenartigen Körpers erzeugbar und so der Relativabstand r zwischen der Hubkurve und dem stangenartigen Körper veränderbar ist.

Bei einer bevorzugten Ausführungsform weist die Vorrichtung eine Vielzahl von Sterilisationseinrichtungen mit Strahlenfingern bzw. stangenartigen Körpern auf, die an einem um eine Rotationsachse E drehbaren gemeinsamen Träger angeordnet sind, sodass sich die einzelnen Strahlenfinger/Sterilisationseinrichtungen entlang eines kreisförmigen (Transport-) Pfades bewegen. Bei der Vorrichtung handelt es sich bevorzugt somit um eine Karussellvorrichtung bzw. der gemeinsame Träger ist in der Art eines Karussells ausgeführt. Weiterhin ist bevorzugt der gemeinsame Träger durch die Bewegungseinrichtung in Längsrichtung L bewegbar. Somit werden alle/die Vielzahl von Sterilisationseinrichtungen durch im Wesentlichen eine einzige Bewegungseinrichtung gegenüber der Hubkurve bewegt, sodass auf Bewegungseinrichtungen für jede einzelne Sterilisationseinrichtung verzichtet werden kann. Hierdurch wird somit vermieden, viele Motoren für die verschiedenen an einem Karussell angeordneten Strahlenfinger/Sterilisationseinrichtungen verwenden zu müssen, da die Strahlenfinger/Sterilisationseinrichtungen entsprechend der vorgeschlagenen Vorrichtung (bzw. dem vorgeschlagenem Verfahren) durch die Bewegung des gesamten Karussells bzw. des gemeinsamen Trägers bewegbar sind.

Bevorzugt ist daher die erzeugte Relativbewegung in der Längsrichtung L zwischen dem stangenartigen Körper bzw. Strahlenfinger und der Hubkurve durch eine Bewegung des Trägers der Vielzahl von Strahlenfingern/Sterilisationseinrichtungen erzeugbar. Die Bewegungseinrichtung ist demnach bevorzugt derart ausgeführt und ausgestaltet, dass die (Relativ-)Bewegung in der Längsrichtung L der Behältnisse zwischen dem stangenartigen Körper und der Hubkurve durch eine Bewegung des Trägers der Vielzahl von Strahlenfingern/stangenartigen Körpern/Sterilisationseinrichtungen erzeugbar und der Relativabstand r zwischen der Hubkurve und dem stangenartigen Körper veränderbar ist.

Somit ist die Vielzahl von Strahlenfingern/Sterilisationseinrichtungen bevorzugt durch den gemeinsamen Träger mechanisch miteinander verbunden und daher sind die Strahlenfingern/Sterilisationseinrichtungen gleich bewegbar, insbesondere drehbar um die Rotationsachse E und in der Längsrichtung L.

Somit ist bevorzugt eine Grundplatte (bzw. der gemeinsame Träger) des gesamten Karussells/der Vorrichtung, auf der die Sterilisationseinrichtungen befestigt sind, in ihrer Höhe veränderbar. So kann beispielsweise ein Behältnis/Vorformling, das/der 20mm kürzer ist behandelt werden, in dem die Grundplatte um 20mm angehoben (somit der Relativabstand r vergrößert) wird. Hierdurch ist eine Endposition des Strahlfingers (der Umkehrpunkt) bezüglich einem Behältnis-/Vorformlingsboden wieder gleich eingestellt, es bleibt demnach der vorbestimmte Abstand a gleich.

Da bei kürzeren Vorformlingen somit die Verweilzeit des Strahlenfingers im Behältnis kürzer ausfällt, wodurch möglicherweise die Sterilisation nicht ausreichend ausgeführt werden könnte, wird bevorzugt vorgeschlagen, eine Geschwindigkeit, mit der das Behältnis/der Vorformling auf einer Flanke der Hubkurve bewegt wird, somit in welcher der Strahlenfinger im Endpunkt in dem Behältnis/Vorformling befindlich ist, zu verlängern. Eine weitere Möglichkeit zur Verbesserung der Sterilisation wäre durch eine Erhöhung der applizierten Dosisleistung, bevorzugt ebenso während das Behältnis/der Vorformling auf einer Flanke der Hubkurve bewegt wird, erreichbar.

Bei einer weiteren bevorzugten Ausführungsform ist der vorbestimmte Abstand während eines Betriebs der Vorrichtung nicht veränderbar. Demnach wird während des Betriebs immer die gleiche Art von Behältnissen sterilisiert, sodass keine Veränderung nötig ist. Wird jedoch auf die Verarbeitung von anderen Behältnissen/(Kunststoff-)Vorformlingen, insbesondere von Behältnissen/(Kunststoff-)Vorformlingen mit unterschiedlicher Länge umgestellt, wird der Relativabstand r zwischen der Hubkurve und dem Strahlenfinger verändert, sodass der vorbestimmte Abstand a zwischen dem Bodenbereich des neu zu verarbeitenden Behältnisses und dem Strahlenfinger wieder angepasst werden kann. Bevorzugt ist der vorbestimmte Abstand a für jegliches Behältnis bzw. jeglichen (Kunststoff-)Vorformling identisch.

Bei einer weiteren bevorzugten Ausführungsform weist der stangenartige Körper bzw. der Strahlenfinger ein Austrittsfenster, für durch die Sterilisationseinrichtung erzeugte Ladungsträger, auf, durch welches die Ladungsträger aus dem stangenartigen Körper austreten und so die Innenwandung der Behältnisse mit den Ladungsträgern beaufschlagt wird. Dieses Austrittsfenster befindet sich, wie aus dem Stand der Technik allgemein bekannt, an der Unterseite des Strahlenfingers, somit an der Seite, welche der Hubkurve am nächsten ist. Weiterhin ist der vorbestimmte Abstand a zwischen einem Bodenbereich der Behältnisse und dem Austrittsfenster des stangenartigen Körpers einstellbar. Vorhergehend ist meist ein Abstand zwischen Strahlenfinger/stangenartigen Körper und einem weiteren Element der Vorrichtung (beispielsweise Hubkurve, Bodenbereich) beschrieben. Hierrunter ist somit bevorzugt der Abstand zwischen dem Austrittsfenster des Strahlenfingers/stangenartigen Körpers und dem jeweiligen weiteren Element zu verstehen. Dieser Abstand ist bevorzugt (im Wesentlichen) in Längsrichtung gegeben bzw. vorliegend.

Bei einer weiteren bevorzugten Ausführungsform sind die Behältnisse über eine jeweilige Halteeinrichtung insbesondere eine Halteklammer, welche das Behältnis unter oder über einem Tragering greift, an einem gemeinsamen Element befestigt. Das gemeinsame Element ist um eine Rotationsachse E drehbar und die jeweilige Halteeinrichtung ist bevorzugt in Längsrichtung (L) beweglich. Die Halteeinrichtung ist somit gegenüber dem gemeinsamen Element in Längsrichtung L beweglich und dementsprechend an diesem angeordnet/befestigt. Bevorzugt ist das gemeinsame Element demnach ein Karussellelement, drehbarer gemeinsamer Träger oder eine drehbare Transporteinrichtung, welche die Behältnisse während der Sterilisation transportiert/bewegt.

Das gemeinsame Element weist bevorzugt eine Vielzahl von Halteeinrichtungen auf, besonders bevorzugt entspricht eine Anzahl der Vielzahl von Halteeinrichtungen der Anzahl der Vielzahl von Sterilisationseinrichtungen, sodass für jede Halteeinrichtung und entsprechend zugehöriges Behältnis an dem gemeinsamen Element eine Sterilisationseinrichtung an dem gemeinsamen Träger vorhanden/angeordnet ist. Bevorzugt ist der vorbestimmte Abstand a erreicht bzw. liegt dieser vor, wenn die Halteeinrichtung oder das Folgeelement (wie etwa eine Kurvenrolle) den höchsten Punkt/Bereich der Hubkurve abfahren bzw. erreichen.

Bevorzugt werden die Sterilisationseinrichtungen sowie die Halteeinrichtungen (bzw. der gemeinsame Träger sowie das gemeinsame Element) um die Rotationsachse E gedreht, welche für beide Einrichtungen identisch ist. Ebenso werden die Einrichtungen mit einer identischen (Dreh-)Geschwindigkeit um diese Rotationsachse E gedreht bzw. bewegt.

Besonders bevorzugt sind der gemeinsame Träger und das gemeinsame Element an einer Achse angeordnet, durch welche die Rotation erzeugbar ist. Es könnte bevorzugt aber auch ein jeweiliger Antrieb bzw. ein jeweiliges Antriebselement für das gemeinsame Element oder den gemeinsamen Träger vorhanden sein.

Bei einer weiteren bevorzugten Ausführungsform weist die Bewegungseinrichtung ein mechanisches Verstellelement, einen hydraulischen oder pneumatischen Antrieb oder einen Elektromotor, insbesondere einen Linearmotor, auf, sodass durch dieses Vorrichtungselement die Bewegung des gemeinsamen Trägers, an dem die Vielzahl von Sterilisationseinrichtungen angeordnet ist, in Längsrichtung L erzeugbar ist.

Durch vorliegende Vorrichtung ist somit bevorzugt eine voneinander unabhängige Bewegung zwischen Strahlenfinger/Sterilisationseinrichtung und den Behältnissen bzw. deren Halteeinrichtung/Klammer oder der Hubkurve ermöglicht/erreichbar. Dabei wird bevorzugt der Strahlenfinger nur in Längsrichtung L bewegt, während kein Betrieb/keine Sterilisation erfolgt, da diese Bewegung zur Einstellung auf das Behältnis oder den Vorformling benötigt wird und nicht für das Sterilisationsverfahren bzw. den Sterilisationsvorgang, da hierfür die Bewegung der Behältnisse bzw. der Halteeinrichtungen aufgrund der Hubkurve genutzt wird bzw. ausreicht.

Bei einer weiteren bevorzugten Vorrichtung ist eine Erkenneinrichtung bzw. Messeinrichtung vorhanden (beispielsweise eine Lasereinrichtung), welche die Länge der jeweiligen zu sterilisierenden Behältnisse erfassen und verarbeiten kann. Weiterhin ist bevorzugt eine Recheneinheit vorhanden, welche derart ausgeführt und ausgestaltet ist, auf den (derzeitigen) Relativabstand r zurückgreifen bzw. diesen verarbeiten und/oder nutzen zu können. Besonders bevorzugt vergleicht und/oder berechnet die Recheneinheit aufgrund des Relativabstands r und der durch die Erkenneinrichtung bzw. Messeinrichtung erfassten Länge, wie weit sich ein Boden des Behältnisses dem Austrittsfenster nähern wird und leitet einen Notstopp der Vorrichtung ein, wenn sich ergibt, dass der Boden des Behältnisses mit dem Austrittsfenster kollidieren oder in Kontakt kommen könnte (bzw. ist die Recheneinheit in genannter Art ausgeführt und ausgestaltet). Anstelle oder neben einem Notstopp könnte auch ein Anheben des Trägers mit den Sterilisationseinrichtungen gegenüber der Hubkurve eingeleitet werden.

Bei einer weiteren vorteilhaften Ausführungsform weist die Vorrichtung einen Reinraum auf, bzw. bildet einen Reinraum aus, innerhalb dessen die Kunststoffbehältnisse während ihrer Sterilisation transportiert werden. Dabei ist bevorzugt dieser Reinraum mittels wenigstens einer Wandung gegenüber einer Umgebung abgedichtet. Bevorzugten wird der Reinraum gegenüber der (unsterilen) Umgebung unter einen Überdruck gesetzt bzw. ist der Reinraum unter einen Überdruck setzbar.

Vorteilhaft bildet auch der besagte gemeinsame Träger der Vielzahl von Sterilisationseinrichtungen, eine Wandung dieses Reinraums aus. Dabei kann bevorzugt nur der Strahlenfinger bzw. stangenartige Körper in den Reinraum reichen oder in diesem angeordnet sein und die weiteren Einheiten der Sterilisationseinrichtung sind außerhalb des Reinraums angeordnet. Bei einer weiteren vorteilhaften Ausführungsform weist die Vorrichtung eine Dichtungseinrichtung auf, um diesen Reinraum gegenüber einer Umgebung abzudichten. Bei dieser Dichtungseinrichtung kann es sich beispielsweise um ein sogenanntes Wasserschloss handeln, welches bevorzugt einen mit einem flüssigen Medium befüllbaren umlaufenden Kanal aufweist, in den ein Wandungselement eintaucht und diesem gegenüber bewegbar ist.
Bei einer weiteren Ausführung wird die Abdichtung des Reinraums mit einem umlaufenden Absaugkanal erzeugt. Dabei wird sowohl aus dem Innenraum bzw. Reinraum und aus dem umgebenden Raum Luft abgesaugt.

Vorteilhaft im Aufbau des Reinraums ist auch die Anordnung der Hubkurve außerhalb des Reinraums. Das Folgeelement incl. Rolle ist dabei außerhalb des Reinraums angeordnet, die Halteeinrichtung ist mittels eines Dichtelements wie Elastomerbalg oder Metallbalg oder ähnlicher reinraumtauglicher Dichtelemente gegenüber der Umgebung abgedichtet.

Bei einer weiteren vorteilhaften Ausführungsform weist die Vorrichtung auch Abschirmelemente auf, um zu verhindern, dass Strahlung, insbesondere Röntgenstrahlung aus der Anlage austritt. Dies können bevorzugt auch Elemente/Wände des Reinraums bzw. der Reinraumgrenze sein.

Bei einer bevorzugten Ausführungsform weist die Sterilisationseinrichtung auch eine Kühleinrichtung zum Kühlen des Austrittsfensters auf. Dabei ist es denkbar, dass der stangenartige Körper/Strahlenfinger einen Kanal aufweist, durch den hindurch ein insbesondere gasförmiges Medium in Richtung des Austrittsfensters geleitet werden kann. Vorteilhaft wird dieser Gasstrom bzw. das gasförmige Medium zum Kühlen des Austrittsfensters wenigstens teilweise und bevorzugt vollständig dem Reinraum entnommen. Vorteilhaft handelt es sich bei dem Austrittsfenster um ein Austrittsfenster aus Titan, welches besonders bevorzugt eine Dicke aufweist, welche zwischen 7 und 13 µm liegt. Bei einer weiteren vorteilhaften Ausführungsform weist die Vorrichtung eine Umlenkungseinrichtung auf, welche insbesondere das gasförmige Medium auf den besagten Bereich der Austrittseinrichtung leitet. Bevorzugt kann es sich bei dem Gasstrom/gasförmigen Medium um einen Luftstrom etwa einen Sterilluftstrom handeln. Es wäre jedoch auch möglich, eine Atmosphäre aus anderen Gasen in dem Reinraum vorzusehen wie etwa eine Stickstoff-Atmosphäre.

Bevorzugt wird die dem Reinraum entnommene Luft (bzw. allgemein das entnommene gasförmige Medium) temperiert und insbesondere gekühlt, bevor sie den einzelnen Austrittsfenstern zugeführt wird. Dabei können zum Kühlen dieser Luft Wärmetauscher, wie an sich aus dem Stand der Technik bekannt, verwendet werden.

Besonders bevorzugt weist die Kühleinrichtung eine erste Beaufschlagungseinrichtung auf, welche einen ersten Bereich des Austrittsfensters mit einem flüssigen Medium kühlt sowie eine zweite Beaufschlagungseinrichtung, welche einen zweiten Bereich des Austrittsfensters mit einem gasförmigen Medium kühlt. Unter einer Kühlung mit dem flüssigen bzw. gasförmigen Medium wird verstanden, dass das Austrittsfenster nicht notwendigerweise selbst mit diesem Medium beaufschlagt werden muss, sondern dass beispielsweise auch Kühlkanäle vorgesehen sein können, durch welche das jeweilige Kühlmedium strömt. Es ist jedoch auch eine direkte Beaufschlagung des Austrittsfensters bzw. eines Bereichs desselben - insbesondere im Hinblick auf das gasförmige Medium - möglich. Grundsätzlich wäre es auch möglich, eine Kühlung der unterschiedlichen Bereiche jeweils mit einem gasförmigen Medium zu erreichen, d. h. beide Bereiche jeweils mittels gasförmigen Mediums zu kühlen.

Bei einer weiteren vorteilhaften Ausführungsform ist eine Kollisionsverhinderungseinrichtung angeordnet, welche einen Kontaktkörper aufweist, der - insbesondere bei Auftreten einer Fehlstellung des Behältnisses und/oder einer Halteeinrichtung zum Halten des Behältnisses - eine Kollision des stangenartigen Körpers mit dem Behältnis, insbesondere der Mündung des Behältnisses verhindert. Damit soll bei dieser Ausführungsform insbesondere eine Kollision des stangenartigen Körpers mit dem Behältnis selbst und/oder dessen Halteeinrichtung verhindert werden.

In der Praxis kann es dazu kommen, dass die Halte- oder Greifelemente, welche die Behältnisse halten, diese nicht ordnungsgemäß zu fassen bekommen und die Behältnisse beispielsweise schief in den Halteelementen liegen. In dieser Situation ist es denkbar, dass bei der Zustellbewegung des Behältnisses zu dem stangenartigen Körper dieser stangenartige Körper mit dem Behältnis kollidiert. Bereits diese Kollision kann ausreichen, um den stangenartigen Körper nachhaltig zu beschädigen.

Durch den besagten Kontaktkörper kann eine derartige Kollision verhindert werden. So ist es möglich, dass die Vorrichtung bei Auftreten eines derartigen mechanischen Kontaktes zwischen dem Behältnis und dem Kontaktkörper die Zustellbewegung anhält. Es wäre jedoch auch möglich, dass in Reaktion auf einen derartigen Kontakt das Halteelement das Behältnis loslässt, sodass dieses nicht mehr mit dem stangenartigen Körper in Kontakt treten kann.

Bevorzugt ist daher dieser Kontaktkörper derart angeordnet, dass der Kontaktkörper das Behältnis kontaktiert, bevor der stangenartige Körper das Behältnis kontaktiert. Bevorzugt weist der Kontaktkörper einen rohrförmigen Körper auf, der den stangenartigen Körper wenigstens abschnittsweise umgibt. Durch diesen rohrförmigen Körper wird der stangenartige Körper einerseits in dessen radialer Richtung gegen Stöße geschützt, andererseits ist es möglich, dass dieser rohrförmige Körper insbesondere schief sitzende Kunststoffbehältnisse berührt, bevor der stangenartige Körper diese berührt.

Bei einer weiteren vorteilhaften Ausführungsform ist eine Schalteinrichtung vorgesehen, welche bewirkt, dass bei einem Kontakt zwischen dem Kontaktkörper und dem Kunststoffbehältnis und/oder der Halteeinrichtung für das Kunststoffbehältnis die Zustellbewegung des Kunststoffbehältnisses angehalten wird. Vorteilhaft ist eine Ausschleuseeinrichtung vorgesehen, welche fehlerhaft an der Halteeinrichtung angeordnete Behältnisse ausschleust.

Die vorliegende Erfindung ist weiterhin auf ein Verfahren zum Sterilisieren von Behältnissen gerichtet, wobei zum Sterilisieren der Behältnisse die Behältnisse über einen stangenartigen Körper geführt werden und im Inneren der Behältnisse eine Innenwandung der Behältnisse mit Strahlung beaufschlagt wird und wobei die Behältnisse in einer Längsrichtung L der Behältnisse bezüglich des stangenartigen Körpers durch eine Hubkurve bewegt werden, um den stangenartigen Körper in das Behältnis durch dessen Mündung einzuführen. Unter einer Führung der Behältnisse über den stangenartigen Körper wird verstanden, dass die stangenartigen Körper zumindest abschnittsweise in die Behältnisse bzw. deren Innenraum eintauchen bzw. eingeführt werden.

Nach dem erfindungsgemäßen Verfahren werden die Hubkurve und der stangenartigen Körper in der Längsrichtung L der Behältnisse zueinander relativ bewegt und ein Relativabstand r zwischen der Hubkurve und dem stangenartigen Körper wird verändert.

Nach einem bevorzugten Verfahren wird der Relativabstand r zwischen der Hubkurve und dem stangenartigen Körper derart verändert, dass ein vorbestimmter Abstand a zwischen einem Bodenbereich der Behältnisse und dem stangenartigen Körper eingestellt wird.

Bevorzugt werden die Behältnisse entlang eines vorgegebenen Transportpfades transportiert, während die Behältnisse sterilisiert werden. Weiterhin bevorzugt ist eine Bewegungseinrichtung angeordnet, welche die Hubkurve und den stangenartigen Körper zueinander relativ bewegt. Besonders bevorzugt bewegt dabei die Bewegungseinrichtung lediglich die stangenartigen Körper bzw. die Sterilisationseinrichtung.

Bei einem weiteren bevorzugten Verfahren ist eine Vielzahl von Sterilisationseinrichtungen mit deren stangenartigen Körpern bzw. Strahlenfingern an einem gemeinsamen Träger angeordnet, der durch die Bewegungseinrichtung gegenüber der Hubkurve in der Längsrichtung L bewegt wird. Bevorzugt wird eine Bewegung der Behältnisse, die über eine Vielzahl von jeweiligen Halteeinrichtungen bzw. Halteklammern an einem gemeinsamen Element angeordnet sind, durch die Ausgestaltung der Hubkurve erreicht.

Des Weiteren erfolgt bevorzugt die Sterilisation in dem Bereich, in dem sich die Hubkurve erhöht, somit könnte eine Aktivierung der Sterilisationseinrichtungen abhängig von deren Position und der Position der Behältnisse aktiviert bzw. deaktiviert werden, sodass die Sterilisationseinrichtung erst aktiviert wird, wenn das Behältnis in einem gewissen Abstand unter dem stangenartigen Körper bzw. dem Strahlenfinger befindlich ist oder erst sobald dieser bereits wenigstens teilweise in dem Behältnis angeordnet ist.

Weitere Vorteile und Ausführungsformen ergeben sich aus den beigefügten Zeichnungen.

Darin zeigen:
Fig. 1 eine schematische Darstellung einer erfindungsgemäßen Vorrichtung zum Sterilisieren von Behältnissen;
Fig. 2 eine weitere schematische Darstellung einer erfindungsgemäßen Vorrichtung zum Sterilisieren von Behältnissen;
Fig. 3 eine schematische Darstellung eines Strahlfingers bzw. stangenartigen Körpers, über den ein Behältnis geführt ist.

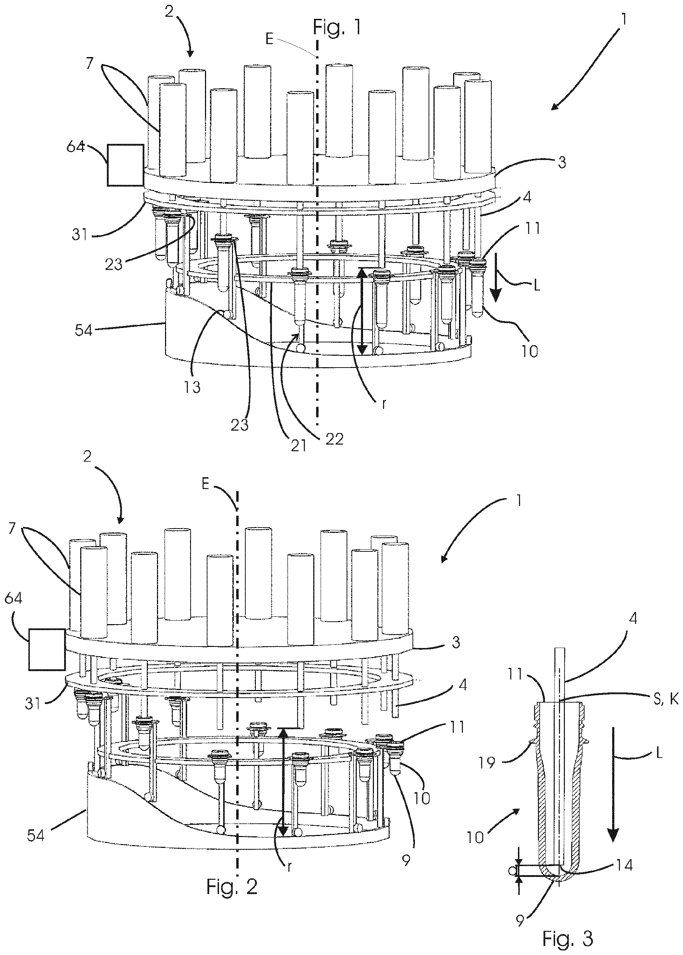

Figur 1 zeigt eine schematische Darstellung einer erfindungsgemäßen Vorrichtung 1 zum Sterilisieren von Behältnissen 10. Wie oben erwähnt, könnte es sich hier jedoch bei den Behältnissen 10 auch (bevorzugt) um Kunststoffvorformlinge 10 handeln. Die Behältnisse 10 erstrecken sich hierbei in einer Längsrichtung L und weisen eine Mündung 11 auf, durch welche hindurch stangenförmige Körper 4 bzw. Strahlenfinger 4 in die Behältnisse geführt werden können. Eine Vielzahl von Behältnissen 10 ist dabei an einem gemeinsamen Element 21 angeordnet. Dieser ist hierbei als Ringträger 21 ausgestaltet, wobei jedoch jegliche Ausgestaltung möglich wäre.

Die Behältnisse 10 werden von Klammern 23 unterhalb deren Trageringe 19 gegriffen bzw. gehalten, wobei die Klammern 23 ein Teilelement einer Halteeinrichtung 22, somit an einer Halteeinrichtung 22 angeordnet, sind, welche wiederum an dem gemeinsamen Element 21 (insbesondere in ihrer Längsrichtung L) beweglich angeordnet, befestigt sind. Am unteren Ende der Halteeinrichtung 22 befindet sich ein Folgeelement 13, wie etwa eine Kurvenrolle.

Dieses Folgeelement 13 läuft auf bzw. folgt einer bevorzugt vorgegebenen, festen Hubkurve 54. Diese Hubkurve 54 ist in der Art ausgestaltet und angeordnet, dass diese eine Auf- und Abbewegung der Folgeelemente 13 und somit der Behältnisse 10 erzeugt bzw. erzeugen kann. Dabei kann in Figur 1 entnommen werden, dass dies durch eine unterschiedliche Höhenausgestaltung der Hubkurve 54 erfolgt. Die Folgeelemente müssen jedoch nicht unbedingt auf der Hubkurve 54 (entlang-) laufen, wie dies in Figur 1 gezeigt ist, es wären auch andere Möglichkeiten für die Hubkurve 54, beispielsweise eine Nut, in der das Folgeelement 13 bewegt wird/läuft, möglich. Weiterhin könnte möglicherweise ein Federelement (nicht gezeigt) angeordnet sein, das das Folgeelement 13 gegenüber oder auf der Hubkurve vorspannt.

Die Bewegung der Behältnisse 10 erfolgt in Figur 1 kreisförmig um eine Rotationsachse E. Es wären jedoch auch andere Bewegungsmöglichkeiten denkbar, wie beispielsweise elliptische oder aber über ein anders ausgestaltetes, gemeinsames Element 21, beispielsweise in der Art einer Transporteinrichtung, welche geradlinige oder freie Bewegungsmöglichkeiten vorgibt. Dementsprechend müsste auch die Hubkurve 54 und die weiteren Elemente (beispielsweise der gemeinsame Träger 3) anders ausgestaltet sein.

Die Vorrichtung 1 weist hierbei weiterhin einen gemeinsamen Träger 3 auf, an welchem eine Vielzahl von Sterilisationseinrichtungen 2, mit den entsprechenden Strahlenfingern bzw. stangenartigen Körpern 4, befestigt/angeordnet ist. Dieser gemeinsame Träger 3 ist dabei auch um die Rotationsachse E drehbar, somit sind die Achsen, um die sich der gemeinsame Träger 3 und das gemeinsame Element 21 drehen, im Wesentlichen deckungsgleich bzw. parallel zueinander und überlappend, sodass sich eine gemeinsame Rotationsachse E ergibt. Mit dem Bezugszeichen 7 sind grob schematisch die weiteren Elemente der Sterilisationseinrichtung 2 angedeutet, welche unter anderem Ladungsträger-/Elektronenerzeuger und Ladungsträger-/Elektronenbeschleuniger sein können.

Weiterhin ist eine Mittel- bzw. Symmetrieachse K des Strahlenfingers (siehe Fig. 3) genauso weit von der Rotationsachse E entfernt, wie eine Mittel- bzw. Symmetrieachse S des Behältnisses 10. Die Behältnisse 10 und Strahlenfinger 4 sind hierbei im Wesentlichen rotationssymmetrisch. Ebenso verlaufen diese beiden Achsen S, K parallel zueinander, was ebenso eine Parallelität zur Längsrichtung L des Behältnisses ergibt. Wie Figur 1 entnommen werden kann, ist die gleiche Anzahl an Sterilisationseinrichtungen 2 am Träger 3, wie auch die gleiche Anzahl an Halteeinrichtungen 22 mit entsprechenden Behältnissen 10 am Element 21, vorhanden bzw. angeordnet. Daraus ergibt sich, dass somit für jedes Behältnis 10 eine Sterilisationseinrichtung 2 vorhanden ist. Die Achsen S und K sind hierbei nicht nur parallel, sondern überlappen einander bzw. sind deckungsgleich, sodass eine möglichst sichere Bewegung/Führung des Behältnisses 10 über die Strahlenfinger 4 erfolgen kann.

Des Weiteren ist diese Längsrichtung L ist hier auch die Beschleunigungsrichtung, in der die Ladungsträger/Elektronen beschleunigt werden und/oder die Bewegungsrichtung, in der sich die Ladungsträger/Elektronen innerhalb des Strahlfingers 4 bevorzugt bewegen.

Mit dem Bezugszeichen 64 ist rein schematisch eine Bewegungseinrichtung gekennzeichnet, welche in dieser Ausführungsform die gesamte "Platte" bzw. den gemeinsamen Träger 3 in Längsrichtung L bewegen, verstellen oder eine Relativbewegung in Längsrichtung L, insbesondere zwischen dem Strahlenfinger 4 und der Hubkurve 54, erzeugen kann. Somit kann durch die Bewegungseinrichtung 64 die Höhe des gemeinsamen Trägers 3 bzw. ein Relativabstand r (welcher hier beispielhaft schematisch mit einem Doppel-Pfeil gekennzeichnet ist) zwischen dem gemeinsamen Träger 3, den Sterilisationseinrichtungen 2 und den Strahlenfingern 4 verändert oder angepasst werden. Der Abstand muss hierbei immer an dem ein und demselben Punkt auf der Hubkurve 54 betrachtet werden, da sich deren Erstreckung in Längsrichtung L ändert. Nach vorliegender Ausführungsform ist daher nicht nur die Erstreckung der Hubkurve in Längsrichtung L fest, sondern ebenso die Anordnung der Hubkurve 54 selbst, die Hubkurve 54 kann demnach bevorzugt nicht in Längsrichtung L bewegt werden.

Die Anordnung der Bewegungseinrichtung 64 ist in Figur 1 nicht einschränkend gewählt und ebenso rein schematisch, beispielsweise könnte eine Welle (nicht gezeigt) vorhanden sein, um die Rotationsachse E vorhanden sein, an welcher sowohl Träger 3 und/oder gemeinsames Element 21 befestigt sind, an welcher ebenso die Bewegungseinrichtung angeordnet oder wenigstens abgestützt ist, sodass über die Bewegungseinrichtung eine Verschiebung/Bewegung in Längsrichtung L des Trägers 3 an dieser Achse erfolgt.

Jedoch könnte die Bewegungseinrichtung 64 auch an einem weiteren Ringelement 31 abgestützt sein, welches fest an der Vorrichtung 1 angeordnet ist. In Figur 1 verlaufen die Strahlenfinger 4 beweglich durch dieses Ringelement 31.

Der linke Bereich der Hubkurve 54 in Figur 1 stellt hierbei den höchsten Punkt dar, den die Behältnisse erreichen können. Somit ist in diesem Bereich der Abstand zwischen einem Behältnisboden 9 und dem Strahlenfinger und insbesondere dessen Austrittsfenster 14 am geringsten. Dies liegt insbesondere im Umkehrpunkt vor, in welchem die Bewegung in Längsrichtung L des Behältnisses 10 (und auf dieses zu) über den Strahlenfinger 4 in eine Bewegung des Behältnisses 10 hinab von dem Strahlenfinger geändert wird. Dabei muss natürlich bedacht werden, dass diese Bewegungsänderung nicht direkt aufeinander erfolgen muss oder kann, sondern das Behältnis 10 über einen gewissen Zeitraum in diesem Umkehrpunkt gehalten werden kann, wie dies insbesondere im linken Bereich der Figur 1 zu sehen ist, in welchem die Hubkurve 54 für einen längeren Abschnitt eine gleichbleibende Erhöhung aufweist, bis diese wieder abnimmt. Dieser Umkehrpunkt, der den geringsten auftretenden Abstand zwischen Austrittsfenster 14 und Behältnisboden 9 darstellt (bzw. im Normalbetrieb darstellen sollte), liegt der vorbestimmte Abstand a zwischen Behältnisboden 9 und Austrittsfenster 14 bzw. Strahlenfinger 4 vor.

In Figur 2 ist im Wesentlichen dieselbe Vorrichtung 1 wie in Figur 1 gezeigt. Jedoch sind hier Behältnisse 10 angeordnet, welche eine kürzere Ausdehnung in Längsrichtung L aufweisen, die somit kürzer sind. Würden diese nun der Vorrichtung 1 nach Figur 1 zugeführt werden, würde der Bodenbereich 9 der Behältnisse 10 mit dem jeweilig eingeführten Strahlenfinger 4 kollidieren, was möglicherweise zu einer Funktionsuntüchtigkeit oder Zerstörung dieses Strahlenfingers 4 und/oder der entsprechenden Sterilisationseinrichtung 2 führen könnte. Daher ist in Figur 2 der gemeinsame Träger 3 weiter von der Hubkurve 54 durch die Bewegungseinrichtung 64 entfernt worden. Es wurde somit der Relativabstand r zwischen Hubkurve 54 und dem Träger 3 bzw. dem Strahlenfinger 4 vergrößert. Hierdurch kann erreicht werden bzw. wurde erreicht, dass der vorbestimmte Abstand a, auch bei dem (im Gegensatz zu Figur 1) kürzeren Behältnis 10, wiederum im Wesentlichen identisch oder gleich ist, wodurch eine Beschädigung der Sterilisationseinrichtung vermieden werden kann sowie dennoch eine ausreichende Sterilisation des Innenraums/der Innenwandung des Behältnisses erreichbar ist.

Die Änderung des Relativabstands r kann auch an der schematischen Bewegungseinrichtung 64 entnommen werden, an welcher sich im Gegensatz zu Figur 1 die Anordnung des gemeinsamen Trägers 3 ebenso verändert hat.

Die Figur 3 zeigt einen Querschnitt eines Behältnisses bzw. eines Vorformlings 10, das über einen Strahlenfinger 4 geführt ist. Hierbei können insbesondere auch die Symmetrieachsen S und K entnommen werden, welche identisch sind/verlaufen und sich parallel zur Längsrichtung L erstrecken. Mit dem Bezugszeichen 14 ist hier am unteren Ende des Strahlenfingers das Austrittsfenster 14 gekennzeichnet, aus welchem die Ladungsträger/Elektronen aus dem Strahlenfinger 4 austreten, um eine Sterilisation der Innenwandung des Vorformlings 10 erzeugen zu können.

Weiterhin ist gut erkennbar, dass sich der Abstand a zwischen Austrittsfenster 14 und dem untersten bzw. mittigsten Punkt des Behältnisbodens 9 erstreckt. Dieser mittigste Punkt ist im Wesentlichen dort, wo die Symmetrieachse S des Behältnisses die Innenwand des Bodenbereichs 9 schneidet. Mit dem Bezugszeichen 19 ist der Tragring des Vorformlings 10 gekennzeichnet unter oder über welchem die Klammer 23 der Halteeinrichtung 22 den Vorformling 10 greift bzw. hält. Ebenso ist die Mündung 11 des Behältnisses 10 ersichtlich.

### Bezugszeichenliste

- 1: Vorrichtung
- 2: Sterilisationseinrichtung
- 3: gemeinsamer Träger
- 4: stangenartiger Körper/Strahlenfinger
- 7: schematische, weitere Elemente der Sterilisationseinrichtung
- 9: Behältnisboden, Vorformlingsboden
- 10: (Kunststoff-)Behältnisse bzw. Vorformlinge
- 11: Mündung
- 13: Folgeelement
- 14: Austrittsfenster
- 19: Tragering
- 21: gemeinsames Element/Ringträger
- 22: Halteeinrichtung
- 23: Klammer
- 31: Ringelement
- 54: Hubkurve
- 64: Bewegungseinrichtung
- a: Abstand zwischen Bodenbereich und Strahlenfinger/Austrittsfenster
- E: Rotationsachse
- L: Längsrichtung
- r: Relativabstand zwischen Hubkurve und Strahlenfinger/Austrittsfenster
- S: Symmetrieachse Behältnis
- K: Symmetrieachse stangenartiger Körper/Strahlenfinger

## Patentansprüche

1. Vorrichtung (1) zum Sterilisieren von Behältnissen (10), mit einer Vielzahl von Sterilisationseinrichtungen (2), die an einem um eine Rotationsachse (E) drehbaren gemeinsamen Träger (3) angeordnet sind, wobei die Sterilisationseinrichtungen einen stangenartigen Körper (4) umfassen, der durch eine Mündung (11) der Behältnisse (10) in einen Innenraum der Behältnisse (10) einführbar ist und welcher eine Innenwandung der Behältnisse (10) mit einer Strahlung beaufschlagt und mit einer Hubkurve (54), welche eine Bewegung in einer Längsrichtung (L) der Behältnisse (10) zwischen den Behältnissen (10) und der Sterilisationseinrichtung (2) derart erzeugt, dass die Behältnisse (10) über den stangenartigen Körper (4) führbar sind,
**dadurch gekennzeichnet, dass**
eine Bewegungseinrichtung (64) vorgesehen ist, welche derart ausgeführt und ausgestaltet ist, dass eine Relativbewegung in der Längsrichtung (L) der Behältnisse (10) zwischen dem stangenartigem Körper (4) und der Hubkurve (54) erzeugbar und ein Relativabstand (r) zwischen der Hubkurve (54) und dem stangenartigen Körper (4) veränderbar ist, wobei der gemeinsame Träger (3) durch die Bewegungseinrichtung (64) in der Längsrichtung (L) der Behältnisse (10) bewegbar ist.

2. Vorrichtung (1) nach Anspruch 1,
**dadurch gekennzeichnet, dass**
die Bewegungseinrichtung (64) derart ausgeführt und ausgestaltet ist, dass eine Relativbewegung in der Längsrichtung (L) der Behältnisse (10) zwischen dem stangenartigem Körper (4) und der Hubkurve (54) erzeugbar und ein Relativabstand (r) zwischen der Hubkurve (54) und dem stangenartigen Körper (4) veränderbar ist, derart, dass ein vorbestimmter Abstand (a) zwischen einem Bodenbereich (9) der Behältnisse (10) und dem stangenartigen Körper (4) einstellbar ist.

3. Vorrichtung (1) wenigstens einem der vorangegangenen Ansprüche,
**dadurch gekennzeichnet, dass**
die Sterilisationseinrichtung (2) und/oder der stangenartige Körper (4) durch die Bewegungseinrichtung (64) in der Längsrichtung (L) der Behältnisse (10) bewegbar ist.

4. Vorrichtung (1) nach wenigstens einem der vorangegangenen Ansprüche,
**dadurch gekennzeichnet, dass**
der vorbestimmte Abstand (a) während eines Betriebs der Vorrichtung nicht veränderbar ist.

5. Vorrichtung (1) nach wenigstens einem der vorangegangenen Ansprüche,
**dadurch gekennzeichnet, dass**
der stangenartige Körper (4) ein Austrittsfenster (14), für durch die Sterilisationseinrichtung erzeugte Ladungsträger, aufweist, durch welches die Ladungsträger aus dem stangenartigen Körper (4) austreten und die Innenwandung der Behältnisse (10) mit den Ladungsträgern beaufschlagt wird und wobei der vorbestimmter Abstand (a) wischen einem Bodenbereich (9) der Behältnisse (10) und dem Austrittsfenster (14) des stangenartigen Körpers (4) einstellbar ist.

6. Vorrichtung (1) nach wenigstens einem der vorangegangenen Ansprüche,
**dadurch gekennzeichnet, dass**
die Behältnisse (10) über eine jeweilige Halteeinrichtung (22) an einem gemeinsamen Element (21) befestigt sind, das um eine Rotationsachse (E) drehbar ist und die jeweilige Halteeinrichtung (21) in Längsrichtung (L) beweglich ist.

7. Vorrichtung (1) nach Anspruch 6,
**dadurch gekennzeichnet, dass**
an jeder Halteeinrichtung (22) ein Folgeelement (13) angeordnet ist, das die Hubkurve abfährt oder dieser folgt, wodurch die Relativbewegung in der Längsrichtung (L) der Behältnisse (10) erzeugbar ist.

8. Vorrichtung (1) nach wenigstens einem der vorangegangenen Ansprüche,
**dadurch gekennzeichnet, dass**
die Bewegungseinrichtung (64) ein mechanisches Verstellelement, einen hydraulischen oder pneumatischen Antrieb oder einen Elektromotor, insbesondere einen Linearmotor, aufweist.

9. Verfahren zum Sterilisieren von Behältnissen (10), wobei zum Sterilisieren der Behältnisse (10) die Behältnisse (10) über einen stangenartiger Körper (4) geführt werden und im Inneren der Behältnisse (10) eine Innenwandung der Behältnisse (10) mit Strahlung beaufschlagt wird und wobei die Behältnisse (10) in einer Längsrichtung (L) der Behältnisse (10) bezüglich des stangenartigen Körpers (4) durch eine Hubkurve (54) bewegt werden, um den stangenartigen Körper (4) in das Behältnis (10) durch dessen Mündung (11) einzuführen,
**dadurch gekennzeichnet, dass**
die Hubkurve (54) und der stangenartige Körper (4) in der Längsrichtung (L) der Behältnisse (10) zueinander relativ bewegt werden und so ein Relativabstand (r) zwischen der Hubkurve (54) und dem stangenartigen Körper verändert wird, wobei eine Vielzahl von Sterilisationseinrichtungen (2) an einem um eine Rotationsachse (E) drehbaren gemeinsamen Träger (3) angeordnet ist und der gemeinsame Träger (3) durch eine Bewegungseinrichtung (64) in der Längsrichtung (L) der Behältnisse bewegt wird.

## Claims

1. An apparatus (1) for the sterilization of containers (10) with a plurality of sterilization devices (2), which are arranged on a common carrier (3) rotatable about an axis of rotation (E), wherein the sterilization devices have a rod-like body (4) which is capable of being introduced through an aperture (11) of the containers (10) into an interior space of the containers (10) and which acts upon an inner wall of the containers (10) with radiation, and with a lifting cam (54) which produces a movement in a longitudinal direction (L) of the containers (10) between the containers (10) and the sterilization device (2) in such a way that the containers (10) are capable of being guided over the rod-like body (4),
**characterized in that**
a movement device (64) is provided which is designed and arranged in such a way that a relative movement in the longitudinal direction (L) of the containers (10) between the rod-like body (4) and the lifting cam (54) is capable of being produced and a relative distance (r) between the lifting cam (54) and the rod-like body (4) is capable of being altered, wherein the common carrier (3) can be moved by the movement device (64) in the longitudinal direction (L) of the containers (10).

2. An apparatus (1) according to claim 1,
**characterized in that**
the movement device (64) is designed and arranged in such a way that a relative movement in the longitudinal direction (L) of the containers (10) is capable of being produced between the rod-like body (4) and the lifting cam (54) and a relative distance (r) between the lifting cam (54) and the rod-like body (4) is capable of being altered, in such a way that a pre-determined distance (a) between a base area (9) of the containers (10) and the rod-like body (4) is capable of being set.

3. An apparatus (1) according to at least one of the preceding claims,
**characterized in that**
the sterilization device (2) and/or the rod-like body (4) is movable by the movement device (64) in the longitudinal direction (L) of the containers (10).

4. An apparatus (1) according to at least one of the preceding claims,
**characterized in that**
the pre-determined distance (a) is not capable of being altered during the operation of the apparatus.

5. An apparatus (1) according to at least one of the preceding claims,
**characterized in that**
the rod-like body (4) has an outlet window (14) for charge carriers produced by the sterilization device, by which outlet window (14) the charge carriers issue from the rod-like body (4), and the inner wall of the containers (10) is acted upon with the charge carriers, and wherein the pre-determined distance (a) between a base area (9) of the containers (10) and the outlet window (14) of the rod-like body (4) is capable of being set.

6. An apparatus (1) according to at least one of the preceding claims,
**characterized in that**
the containers (10) are fastened by way of a respective holding device (22) to a common element (21) which is rotatable about an axis of rotation (E) and the respective holding device (21) is movable in the longitudinal direction (L).

7. An apparatus (1) according to claim 6,
**characterized in that**
a follower element (13) which runs on the lifting cam or follows it is arranged on each holding device (22), as a result of which the relative movement in the longitudinal direction (L) of the containers (10) is capable of being produced.

8. An apparatus (1) according to at least one of the preceding claims,
**characterized in that**
the movement device (64) has a mechanical displacement element, an hydraulic or pneumatic drive or an electric motor, in particular a linear motor.

9. A method for the sterilization of containers (10), wherein in order to sterilize the containers (10) the containers (10) are conveyed over a rod-like body (4) and in the interior of the containers (10) an inner wall of the containers (10) is acted upon with radiation and wherein the containers (10) are moved by a lifting cam (54) with respect to the rod-like body (4) in a longitudinal direction (L) of the containers (10) in order to introduce the rod-like body (4) into the container (10) through the aperture (11) thereof,
**characterized in that**
the lifting cam (54) and the rod-like body (4) are moved relative to each other in the longitudinal direction (L) of the containers (10) and a relative distance (r) between the lifting cam (54) and the rod-like body is altered in this way, wherein a plurality of sterilizing devices (2) is arranged on a common carrier (3) rotatable about an axis of rotation (E) and the common carrier (3) is moved in the longitudinal direction (L) of the containers by a movement device (64).

## Revendications

1. Dispositif (1) servant à stériliser des récipients (10), comprenant une pluralité de systèmes de stérilisation (2), qui sont disposés au niveau d'un support (3) commun pouvant tourner autour d'un axe de rotation (E), dans lequel les systèmes de stérilisation comprennent un corps (4) de type tige, qui peut être introduit par une embouchure (11) des récipients (10) dans un espace intérieur des récipients (10) et qui soumet une paroi intérieure des récipients (10) à l'action d'un rayonnement, et comprenant une came de levage (54), qui génère un déplacement dans une direction longitudinale (L) des récipients (10) entre les récipients (10) et le système de stérilisation (2) de telle manière que les récipients (10) peuvent être guidés au-dessus du corps (4) de type tige,
**caractérisé en ce que**
un système de déplacement (64) est prévu, lequel est réalisé et est configuré de telle manière qu'un déplacement relatif dans la direction longitudinale (L) des récipients (10) peut être généré entre le corps (4) de type tige et la came de levage (54) et qu'un espacement relatif (r) entre la came de levage (54) et le corps (4) de type tige peut être modifié, dans lequel le support (3) commun peut être déplacé par le système de déplacement (64) dans la direction longitudinale (L) des récipients (10).

2. Dispositif (1) selon la revendication 1,
**caractérisé en ce que**
le système de déplacement (64) est réalisé et configuré de telle manière qu'un déplacement relatif dans la direction longitudinale (L) des récipients (10) peut être généré entre le corps (4) de type tige et la came de levage (54) et qu'un espacement relatif (r) entre la came de levage (54) et le corps (4) de type tige peut être modifié de telle manière qu'un espacement (a) prédéfini entre la zone de fond (9) des récipients (10) et le corps (4) de type tige peut être réglé.

3. Dispositif (1) selon au moins l'une quelconque des revendications précédentes,
**caractérisé en ce que**
le système de stérilisation (2) et/ou le corps (4) de type tige peuvent être déplacés par le système de déplacement (64) dans la direction longitudinale (L) des récipients (10).

4. Dispositif (1) selon au moins l'une quelconque des revendications précédentes,
**caractérisé en ce que**
l'espacement (a) prédéfini ne peut pas être modifié pendant un fonctionnement du dispositif.

5. Dispositif (1) selon au moins l'une quelconque des revendications précédentes,
**caractérisé en ce que**
le corps (4) de type tige présente une fenêtre de sortie (14) pour des supports de charge générés par le système de stérilisation, par laquelle les supports de charge sortent hors du corps (4) de type tige, et la paroi intérieure des récipients (10) est soumise à l'action des supports de charge, et dans lequel l'espacement (a) prédéfini entre une zone de fond (9) des récipients (10) et la fenêtre de sortie (14) du corps (4) de type tige peut être réglé.

6. Dispositif (1) selon au moins l'une quelconque des revendications précédentes,
**caractérisé en ce que**
les récipients (10) sont fixés, par l'intermédiaire d'un système de maintien (22) respectif, au niveau d'un élément commun (21), qui peut être tourné autour d'un axe de rotation (E), et le système de maintien (21) respectif est mobile dans la direction longitudinale (L).

7. Dispositif (1) selon la revendication 6,
**caractérisé en ce que**
est disposé, au niveau de chaque système de maintien (22), un élément suiveur (13), qui s'écarte de la came de levage ou la suit, ce qui permet de générer le déplacement relatif dans la direction longitudinale (L) des récipients (10).

8. Dispositif (1) selon au moins l'une quelconque des revendications précédentes,
**caractérisé en ce que**
le système de déplacement (64) présente un élément d'ajustement mécanique, un entraînement hydraulique ou pneumatique ou un moteur électrique, en particulier un moteur linéaire.

9. Système servant à stériliser des récipients (10), dans lequel les récipients (10) sont guidés au-dessus d'un corps (4) de type tige afin de stériliser les récipients (10), et une paroi intérieure des récipients (10) est soumise à l'action d'un rayonnement à l'intérieur des récipients (10) et dans lequel les récipients (10) sont déplacés par une came de levage (54) dans une direction longitudinale (L) des récipients (10) par rapport au corps (4) de type tige afin d'introduire le corps (4) de type tige dans le récipient (10) à travers son embouchure (11),
**caractérisé en ce que**
la came de levage (54) et le corps (4) de type tige sont déplacés l'un par rapport à l'autre dans la direction longitudinale (L) des récipients (10), et ainsi un espacement relatif (r) entre la came de levage (54) et le corps de type tige est modifié, dans lequel une pluralité de systèmes de stérilisation (2) est disposée au niveau d'un support (3) commun pouvant tourner autour d'un axe de rotation (E) et le support (3) commun est déplacé par un système de déplacement (64) dans la direction longitudinale (L) des récipients.
